# EUROPEAN PATENT APPLICATION

(11) **EP 4 620 522 A2**
(43) Date of publication of application: **24.09.2025**
(21) Application number: 25194517.6
(22) Date of filing: 05.08.2020
(51) Int. Cl.: A61P 3/10

(54) **A COMBINATION COMPRISING VILDAGLIPTIN AND METFORMIN**

(30) Priority: 06.09.2019 TR 201913472
(62) Divisional of application: 20860939.6
(71) Applicant: Sanovel Ilac Sanayi ve Ticaret A.S., 34394 Istanbul (TR)
(72) Inventor: PALANTOKEN, Arzu, 34394 Istanbul (TR); TOMBAYOGLU, Vildan, 34394 Istanbul (TR); SUNEL, Fatih, 34394 Istanbul (TR); MUTLU, Onur, 34394 Istanbul (TR)
(74) Representative: Bakirci, Utkan Bahri

(57) **Abstract**

The present invention relates to a solid oral pharmaceutical composition comprising vildagliptin, metformin or pharmaceutically acceptable salt thereof with a coating which is free of hydroxypropylmethyl cellulose.

## Description

### Field of the invention

The present invention relates to a solid oral pharmaceutical composition comprising vildagliptin, metformin or pharmaceutically acceptable salt thereof with a coating which is free of hydroxypropylmethyl cellulose or hydroxypropyl cellulose.

### Background of the invention

Diabetes mellitus is a group of disorders of carbohydrate metabolism in which the action of insulin is diminished or absent through altered secretion, decreased insulin activity or a combination of both factors. There are two main types of diabetes; Type 1 and Type 2:
Type 1 diabetes occurs because the insulin-producing cells of the pancreas (beta cells) are damaged. In Type 1 diabetes, the pancreas makes little or no insulin, so sugar cannot get into the body's cells for use as energy. People with Type 1 diabetes must use insulin injections to control their blood glucose.

In Type 2 diabetes, the pancreas makes insulin, but it either doesn't produce enough, or the insulin does not work properly. This diabetes occurs most often in people who are over 40 years old and overweight. Type 2 diabetes may sometimes be controlled with a combination of diet, weight management, and exercise. However, treatment also may include oral glucose-lowering medications or insulin injections.

Metformin is antidiabetics having an orally-administrated biguanide structure. Metformin hydrochloride is a white to off-white crystalline compound and it is freely soluble in water and practically insoluble in acetone, ether, and chloroform. Oral doses of metformin are generally recommended in the range of 500 to 2500 mg a day and a single dose may vary from 250 to 1000 mg. It is used singly or in combination with sulfonylureas, alpha-glucosidase inhibitors, or insulin.

The chemical name of metformin is 1,1-dimethyl biguanide, has the following chemical structure of Formula I.

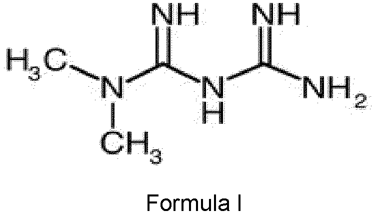

Although metformin is effective at lowering blood glucose levels, its use is associated with gastrointestinal (GI) adverse effects, particularly diarrhea and nausea. These adverse effects may limit the tolerated dose of metformin and cause patients to discontinue the therapy. Also, it does not promote insulin secretion. Its hypoglycemic effect is mainly to promote the uptake of glucose by adipose tissue, increase the anaerobic glycolysis of muscle tissue, increase the utilization of glucose, and reduce the absorption of glucose through the digestive tract.

Both vildagliptin and metformin hydrochloride are effective in the treatment of diabetes, and formulations containing both drugs as active ingredients are highly useful for clinical use.

Vildagliptin is a dipeptidyl dipeptidase-IV (DPP-IV) inhibitor developed for use in the treatment of type 2 diabetes (non-insulin dependent diabetes). Vildagliptin inhibits the degradation of the dipeptidyl dipeptidase-IV enzyme, thereby inhibiting the effects of incretin hormones, glucagon-like peptide-1 (GLP-1), and of glucose-dependent insulinotropic peptide (GIP). The chemical designation of vildagliptin is (S)-{[(3-hydroxyadamantan-1-yl)amino]acetyl}pyrrolidine-2-carbonitril, with the chemical structure illustrated below in Formula II.

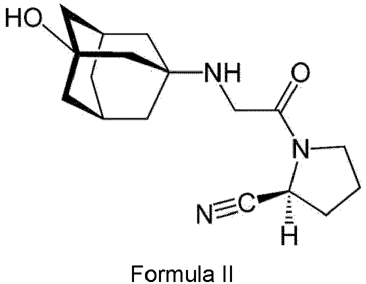

Vildagliptin is an active agent that is highly-susceptible to air and humidity conditions. When vildagliptin is initially exposed to air and humidity, it degrades structurally and develops chemical behavioral changes. As a result of this, two main problems emerge. The first problem is that the stability of the products developed is not at a desired level and the shelf life thereof is shortened. Secondly, vildagliptin is reactive against the excipients employed in developing formulations. This fact causes impurities and unwanted components to be included into the formulation.

Combination product of vildagliptin and metformin hydrochloride is marketed under the trademark Eucreas^{®} (50mg/850mg and 50mg/1000mg dosage forms of vildagliptin and metformin hydrochloride).

Because of the aforementioned highly-susceptible to air and humidity conditions of vildagliptin, a developing formulation for the combination of vildagliptin and metformin must contain a coating. In the prior art there are already known documents on the subject.

Coatings are generally applied to tablets or capsules to protect the ingredients against the atmosphere (humidity, temperature, light), to mask unpleasant tastes and odors, to improve the appearance as well as providing for coloring and printing.

EP1948149(A2) patent application discloses a formulation comprising vildagliptin and metformin, to tablets comprising such formulations and to processes for the preparation thereof. The formulation comprises hydroxylpropylmethyl cellulose and hydroxylpropylmethyl cellulose in a coating or composition.

EP3086781(A1) patent application discloses a formulation comprising vildagliptin and metformin which can be produced by wet granulation and use of standard pharmaceutical excipients and coating.

In recent years, cellulose derivatives, especially hydroxypropylmethyl cellulose or hydroxypropyl cellulos, have gained a wide acceptance as coating materials for pharmaceutical forms. The coatings of the prior art, however, are usually tacky, uneven, require extensive polishing after coating, and are damaged when an attempt is made to print the coated pharmaceutical form employing conventional printing techniques. Also, it should be mentioned that cellulose derivatives are used in a coating, when coming into contact with the atmosphere, they take up small amounts of moisture. Unsuccessful attempts have been made to overcome said disadvantages of cellulosic coating in a composition comprising vildagliptin, and it is with the alleviation of these disadvantages that the present invention is primarily concerned.

In addition, HPMC and HPC have high viscosity excipients. When used in the composition, this presents some disadvantages. In particular, its use with active ingredients having compressibility problems such as metformin raises some problems for the tablet or capsule form.

Therefore, remains a need for a stable pharmaceutical composition comprising vildagliptin and metformin whose manufacturing process is simple, reliable, straightforward, economical and may be conducted using standard manufacturing methods and raw materials.

Attempts to overcome these difficulties through the teachings of the prior art were unsatisfactory, and further innovation was necessary to achieve the desired results.

### Detailed description of the Invention

The main object of the present invention is to provide a solid oral pharmaceutical composition of a combination of vildagliptin, metformin or pharmaceutically acceptable salt thereof having high chemical and physical stability. For the stability, the composition comprises the coating which is free of hydroxypropylmethyl cellulose or hydroxypropyl cellulose.

Another object of the present invention is to provide inexpensive and efficient methods of the combination comprising vildagliptin, metformin or pharmaceutically acceptable salt thereof having desired compressibility and flowability by the help of selection of excipients.

Another object of the present invention is to provide desired dissolution profile in the composition which does not degrade to unwanted impurities.

The term "metformin" as used throughout the specification refers to not only metformin, but also its other pharmaceutically acceptable salt, pharmaceutically acceptable solvates, pharmaceutically acceptable hydrates thereof.

According to one embodiment of the present invention, a solid oral pharmaceutical composition comprises vildagliptin, metformin or pharmaceutically acceptable salt thereof with a coating; wherein, the coating is free of hydroxypropylmethyl cellulose or hydroxypropyl cellulose.

Using hydroxypropylmethyl cellulose or hydroxypropyl cellulose in coating, when coming into contact with the atmosphere, they take up small amounts of moisture. Thus, this is undesirable in a solid composition. So, the coating is free of hydroxypropylmethyl cellulose or hydroxypropyl cellulose. This provides desired stability of the composition.

According to one embodiment of the present invention, coating is film coating or enteric coating. Preferably, coating is film coating.

According to one embodiment of the present invention, preferably, used metformin in the composition is present as having pharmaceutically acceptable salts thereof. More preferably, metformin is present as metformin hydrochloride.

According to one embodiment of the present invention, the amount of vildagliptin in the composition is 1.0% to 15.0%, 1.0% to 10.0%, 3.0% to 8.0%, 3.0% to 5.0 by weight.

According to one embodiment of the present invention, the amount of metformin in the composition is 66.0% to 85.0%, 70.0% to 80.0%, 72.0% to 79.0%, 73.0% to 76.0 by weight.

According to one embodiment of the present invention, the composition further comprises at least one pharmaceutically acceptable excipients which are selected from fillers, disintegrants, binders, lubricants or mixtures thereof.

Suitable fillers are selected from group comprising microcrystalline cellulose, anhydrous dibasic calcium phosphate, pregelatinized starch, lactose monohydrate, starch, mannitol, tribasic calcium phosphate, trehalose, isomalt, sodium carbonate, sodium bicarbonate, calcium carbonate or mixtures thereof.

According to one embodiment of the present invention, the filler is microcrystalline cellulose or anhydrous dibasic calcium phosphate or pregelatinized starch or mixtures thereof.

According to one embodiment of the present invention, the amount of fillers in the composition is 5.0% to 20.0%, 7.0% to 15.0%, 8.0% to 13.0% by weight. This ratio helps the compressibility of metformin.

Suitable disintegrants are selected from group comprising croscarmellose sodium, docusate sodium, guar gum, alginates, ion-exchange resins, sodium starch glycolate, calcium silicate, sodium glycine carbonate or mixtures thereof.

According to one embodiment of the present invention, the disintegrant is croscarmellose sodium.

According to one embodiment of the present invention, the amount of disintegrants in the composition is 0.5% to 10.0%, 0.5% to 8.0%, 1.0% to 5.0% by weight.

Suitable binders are selected from group comprising polyvinylpyrrolidone, sugars, lactose, natural gums, carbomers, carboxymethylcellulose sodium, chitosan, copovidone, starch, corn starch, glyceryl behenate, hydrogenated vegetable oil type I, hydroxypropyl starch, magnesium aluminum silicate, maltodextrin, maltose, pectin, poloxamer, polycarbophil, polydextrose, polyethylene oxide, polymethacrylates, aluminia hydroxide, bentonite, cetostearyl alcohol, polyoxyethilene-alkyl ethers, pullulan or mixtures thereof.

According to one embodiment of the present invention, the amount of binders in the composition is 1.0% to 10.0%, 1.0% to 8.0%, 2.0% to 5.0% by weight.

Suitable lubricants are selected from the group comprising magnesium stearate, colloidal silicone dioxide, calcium stearate, sodium stearyl fumarate, potassium stearate, stearic acid, sodium chloride, sodium benzoate, sodium acetate, sodium oleate, polyethylene glycols or mixtures thereof.

According to one embodiment of the present invention, the lubricant is magnesium stearate or colloidal silicone dioxide or mixtures thereof.

According to one embodiment of the present invention, the amount of lubricants in the composition is 1.0% to 10.0%, 1.0% to 8.0%, 2.0% to 5.0% by weight.

According to one embodiment of the present invention, the solid oral pharmaceutical composition is free of hydroxypropylmethyl cellulose or hydroxypropyl cellulose.

According to another embodiment of the present invention, the solid oral pharmaceutical composition comprises vildagliptin, metformin or pharmaceutically acceptable salts thereof with a coating; wherein, the coating is free of hydroxypropylmethyl cellulose.

According to a preferred embodiment the solid oral pharmaceutical composition is free of hydroxypropylmethyl cellulose.

Vildagliptin is reactive against the excipients employed in developing formulations. But, in the present invention, vildagliptin did not react with the above-mentioned excipients. Vildagliptin-induced impurities have not occurred, particularly due to the lack of hydroxypropylmethyl cellulose or hydroxypropyl cellulose.

According to one embodiment of the present invention, the excipients are added to the composition to impart good flow and compression characteristics to the material being compressed. Such properties are imparted to these excipients through pretreatment steps such as wet granulation, slugging, direct compression. The method of preparation and type of excipients are selected to give the tablet formulation the desired physical characteristics that allow for the rapid compression of the tablets.

According to one embodiment of the present invention, the solid oral pharmaceutical composition is in the form of tablet or capsule. Preferably, the composition is in the form of tablet.

Metformin HCl is hygroscopic, presents stability problems, and is not inherently compressible. Consequently, there is a need to provide a free-flowing and cohesive metformin HCl composition capable into strong tablets with an acceptable in dissolution profile. In the present invention, the composition is free of HPMC and HPC, because of having high viscosity of HPMC and HPC. So, metformin can be compressed into a solid oral pharmaceutical composition more easily.

According to one embodiment of the present invention, the solid oral pharmaceutical composition has low friability, good compactness, acceptable degree of hardness as well as acceptable dissolution profile and proper disintegration time. This is achieved by preferred excipients in coating and formulation and by the production methods of the composition.

The combination preparations described in the prior art have the drawback that the tablets can be produced by wet granulation, as the active substance metformin is a very poorly compressible active substance. In wet granulation process there is, however, the risk that the active substance might be decomposed through interactions with the solvent used, that causes formation of undesirable degradation products.

Suitable solvents are selected from the group comprising pure water, isopropyl alcohol, propylene glycol, polyethylene glycol, glycerin, ethyl alcohol or mixtures thereof.

According to one embodiment of the present invention, the coating comprises at least one coating. The coating provides desired dissolution profile and stability.

Suitable coating agent are selected from the group comprising polymethacrylates, lactose monohydrate, polyvinyl alcohol (PVA), polyethylene glycol (PEG), talc, polyvinyl alcohol-polyethylene glycol copolymers (Kollicoat^{®} IR), ethylcellulose dispersions (Surelease^{®}), polyvinylprolidone, polyvinylprolidone-vinyl acetate copolymer (PVP-VA), pigments, dyes, titanium dioxide, iron oxide or mixtures thereof.

### Example 1: A solid oral pharmaceutical composition comprising vildagliptin and metformin HCl

| **Ingredients** | **Amount (% by weight of the total)** |
|---|---|
| Vildagliptin | 1.0 - 15.0 |
| Metformin HCl | 66.0 - 85.0 |
| Fillers | 5.0 - 20.0 |
| Binders | 1.0-10.0 |
| Disintegrant | 0.5 - 10.0 |
| Lubricant | 0.05 - 5.0 |

| **Core tablet** | |
|---|---|
| Coating agents (free of hydroxypropylmethyl cellulose or hydroxypropyl cellulose) | 1.0 - 5.0 |
| **Total coated tablet** | **100** |

### Example 2: A tablet comprising vildagliptin and metformin HCl processed with slugging

| **Ingredients** | **Amount (% by weight of the total)** |
|---|---|
| Vildagliptin | 2.0 - 10.0 |
| Metformin HCl | 68.0 - 80.0 |
| Microcrystalline cellulose and anhydrous dibasic calcium phosphate | 7.0 - 15.0 |
| Polyvinylpyrrolidone | 2.0 - 8.0 |
| Croscarmellose sodium | 0.5 - 5.0 |
| Colloidal silicone dioxide | 0.5 - 5.0 |
| Magnesium stearate | 0.05 - 2.0 |

| **Core tablet** | |
|---|---|
| Coating agents (free of hydroxypropylmethyl cellulose or hydroxypropyl cellulose) | 1.0 - 5.0 |
| **Total coated tablet** | **100** |

### Process for example 2;

A process for preparing the tablet in example 2 comprises the following steps:
a) Adding vildagliptin, metformin HCl, microcrystalline cellulose and anhydrous dibasic calcium phosphate, polyvinylpyrrolidone, colloidal silicone dioxide and then, mixing
b) Compressing the mixture into a tablet having hardness of 50N and breaking
c) Adding croscarmellose sodium and then, mixing
d) Adding magnesium stearate
e) Compressing the mixture into a tablet
f) Final tablet is coated with coating agents.

### Example 3: A tablet comprising vildagliptin and metformin HCl processed with direct compression

| **Ingredients** | **Amount (% by weight of the total)** |
|---|---|
| Vildagliptin | 2.0 - 10.0 |
| Metformin HCl | 68.0 - 80.0 |
| Pregelatinized starch | 7.0 - 15.0 |
| Polyvinylpyrrolidone | 2.0 - 8.0 |
| Croscarmellose sodium | 0.5 - 5.0 |
| Colloidal silicone dioxide | 0.5 - 5.0 |
| Magnesium stearate | 0.05 - 2.0 |

| **Core tablet** | |
|---|---|
| Coating agents (free of hydroxypropylmethyl cellulose or hydroxypropyl cellulose) | 1.0 - 5.0 |
| **Total coated tablet** | **100** |

### A method with direct compression

A process for preparing the tablet in example 3 comprises the following steps:
a) Adding vildagliptin, metformin HCl, pregelatinized starch, polyvinylpyrrolidone, colloidal silicone dioxide
b) Adding croscarmellose sodium and then, mixing
c) Adding magnesium stearate
d) Compressing the mixture into a tablet.
e) Final tablet is coated with coating agents.

### Example 4: A tablet comprising vildagliptin and metformin HCl processed with wet granulation

| **Ingredients** | **Amount (% by weight of the total)** |
|---|---|
| Vildagliptin | 2.0 - 10.0 |
| Metformin HCl | 68.0 - 80.0 |
| At least one binder | 1.0 - 10.0 |
| Microcrystalline cellulose and anhydrous dibasic calcium phosphate | 7.0 - 15.0 |
| Croscarmellose sodium | 0.5 - 5.0 |
| Ethyl alcohol | q.s. |
| Magnesium stearate | 0.05 - 2.0 |

| **Core tablet** | |
|---|---|
| Coating agents (free of hydroxypropylmethyl cellulose or hydroxypropyl cellulose) | 1.0 - 5.0 |
| **Total coated tablet** | **100** |

| | |
|---|---|
| **q.s.: quantity sufficient** | |

### A method with wet granulation

A process for preparing the tablet in example 4 comprises the following steps:
a) Adding metformin HCl, microcrystalline cellulose and anhydrous dibasic calcium phosphate in a tank
b) Dissolving at least one binder with ethyl alcohol in a separate tank,
c) Adding step (b) mixture to step (a) mixture,
d) Drying the final mixture, then sieving it,
e) Adding vildagliptin and croscarmellose sodium and then, mixing
f) Adding magnesium stearate
g) Compressing the mixture into a tablet
h) Final tablet is coated with coating agents.

## Claims

1. A solid oral pharmaceutical composition comprising vildagliptin, metformin or pharmaceutically acceptable salts thereof with a coating; wherein, the coating is free of hydroxypropylmethyl cellulose.

2. The solid oral pharmaceutical composition according to claim 1, wherein metformin or pharmaceutically acceptable salts thereof is present as metformin hydrochloride.

3. The solid oral pharmaceutical composition according to claim 1, wherein the amount of vildagliptin in the composition is 1.0% to 15.0%, 1.0% to 10.0%, 3.0% to 8.0% or 3.0% to 5.0 by weight.

4. The solid oral pharmaceutical composition according to claim 2, wherein the amount of metformin hydrochloride in the composition is 66.0% to 85.0%, 70.0% to 80.0%, 72.0% to 79.0% or 73.0% to 76.0 by weight.

5. The solid oral pharmaceutical composition according to claim 1, further comprising at least one the pharmaceutically acceptable excipients which are selected from fillers, disintegrants, binders, lubricants or mixtures thereof.

6. The solid oral pharmaceutical composition according to claim 5, wherein the amount of fillers in the composition is 5.0% to 20.0%, 7.0% to 15.0%, 8.0% to 13.0% by weight.

7. The solid oral pharmaceutical composition according to claim 6, fillers are selected from group comprising microcrystalline cellulose, anhydrous dibasic calcium phosphate, pregelatinized starch, lactose monohydrate, starch, mannitol, tribasic calcium phosphate, trehalose, isomalt, sodium carbonate, sodium bicarbonate, calcium carbonate or mixtures thereof.

8. The solid oral pharmaceutical composition according to claim 7, wherein the filler is microcrystalline cellulose or anhydrous dibasic calcium phosphate or pregelatinized starch or mixtures thereof.

9. The solid oral pharmaceutical composition according to claim 5, wherein disintegrants are selected from group comprising croscarmellose sodium, docusate sodium, guar gum, alginates, ion-exchange resins, sodium starch glycolate, calcium silicate, sodium glycine carbonate or mixtures thereof.

10. The solid oral pharmaceutical composition according to claim 9, wherein the disintegrant is croscarmellose sodium.

11. The solid oral pharmaceutical composition according to claim 5, wherein binders are selected from group comprising polyvinylpyrrolidone, sugars, lactose, natural gums, carbomers, carboxymethylcellulose sodium, chitosan, copovidone, starch, corn starch, glyceryl behenate, hydrogenated vegetable oil type I, hydroxypropyl starch, magnesium aluminum silicate, maltodextrin, maltose, pectin, poloxamer, polycarbophil, polydextrose, polyethylene oxide, polymethacrylates, aluminia hydroxide, bentonite, cetostearyl alcohol, polyoxyethilene-alkyl ethers, pullulan or mixtures thereof.

12. The solid oral pharmaceutical composition according to any preceding claim, wherein the composition is in the form of tablet or capsule; preferably, is in the form of tablet.

13. The solid oral pharmaceutical composition according to any preceding claim, wherein the composition is free of hydroxypropylmethyl cellulose.

14. The solid oral pharmaceutical composition according to any preceding claim, the composition comprising;
a) 1.0 - 15.0% by weight of vildagliptin
b) 66.0 - 85.0% by weight of metformin HCl
c) 5.0 - 20.0% by weight of microcrystalline cellulose or anhydrous dibasic calcium phosphate or pregelatinized starch or mixtures thereof
d) 0.5 - 10.0% by weight of croscarmellose sodium
e) 1.0 - 10.0% by weight of at least one binder
f) 0.05 - 5.0% by weight of magnesium stearate or colloidal silicone dioxide or mixtures thereof
g) 1.0 - 5.0% by weight of coating agents in the composition.

15. The solid oral pharmaceutical composition according to claim 14, wherein the composition is processed by wet granulation, slugging or by direct compression.
